# EUROPEAN PATENT SPECIFICATION

(11) **EP 2 750 680 B1**
(45) Date of publication and mention of the grant of the patent: **26.12.2018**
(21) Application number: 12828991.5
(22) Date of filing: 28.08.2012
(51) Int. Cl.: A01N 31/04, A01N 35/02, A01N 37/02, A01N 37/36, A01P 7/02, A61K 9/107, A61K 47/32

(54) **COMPOSITION FOR TREATING LICE INFESTATION**
ZUSAMMENSETZUNG ZUR BEKÄMPFUNG EINES LÄUSEBEFALLS
COMPOSITION POUR TRAITER UNE INFESTATION PAR DES POUX

(30) Priority: 29.08.2011 US 201161528318 P
(43) Date of publication of application: 09.07.2014
(73) Proprietor: Levin, Orna, 4059300 Kfar Netter (IL); Forman, Yochanan, 4023000 Kibbutz Maabarot (IL); Royz, Michael, 76382 Rehovot (IL)
(72) Inventor: Levin, Orna, 4059300 Kfar Netter (IL); Forman, Yochanan, 4023000 Kibbutz Maabarot (IL); Royz, Michael, 76382 Rehovot (IL)
(74) Representative: Harrison IP Limited
(86) International application number: PCT/IB2012/054416
(87) International publication number: WO 2013/030768

(56) References cited:
- EP-A2- 2 048 944
- WO-A1-00/62613
- WO-A1-96/14046
- WO-A2-03/092583
- WO-A2-2007/085899
- WO-A2-2008/011054
- WO-A2-2011/023887
- WO-A2-2011/023887
- GB-A- 2 462 526
- IL-A- 84 701
- IL-A- 84 701
- US-A1- 2002 025 336
- US-A1- 2008 299 064
- US-A1- 2008 299 064
- TERRI L. MEINKING ET AL: "The Clinical Trials Supporting Benzyl Alcohol Lotion 5% (Ulesfia TM ): A Safe and Effective Topical Treatment for Head Lice (Pediculosis Humanus Capitis)", PEDIATRIC DERMATOLOGY, vol. 27, no. 1, 1 January 2010 (2010-01-01), pages 19-24, XP055146030, ISSN: 0736-8046, DOI: 10.1111/j.1525-1470.2009.01059.x
- I. F. BURGESS: "Do nit removal formulations and other treatments loosen head louse eggs and nits from hair?", MEDICAL AND VETERINARY ENTOMOLOGY, vol. 24, no. 1, 1 March 2010 (2010-03-01), pages 55-61, XP055177573, ISSN: 0269-283X, DOI: 10.1111/j.1365-2915.2009.00845.x
- BURGESS IAN F.: 'Current treatments for pediculosis capitis' CURRENT OPINION AND INFECTIOUS DISEASES vol. 22, 01 April 2009, pages 131 - 136, XP008172359
- TERRI L. MEINKING ET AL.: 'The Clinical Trials Supporting Benzyl Alcohol Lotion 5% (UlesfiaTM): A Safe and Effective Topical Treatment for Head Lice (Pediculosis Humanus Capitis)' PEDIATRIC DERMATOLOGY vol. 27, no. 1, 01 February 2010, pages 19 - 24, XP055146030

## Description

### RELATED APPLICATION

The present application gains priority from U.S. Provisional Patent Application No. 61/528,318 filed 29 August 2011.

### FIELD AND BACKGROUND OF THE INVENTION

The invention, in some embodiments, relates to the field of pest control, and more specifically, but not exclusively, to compositions for treating infestations by insects, especially infestations by lice (pediculosis).

Lice belong to the group of external parasites living on warm-blooded animals. An infestation of lice is called pediculosis. In humans there are three different varieties of pediculosis caused by different types of lice: (1) Pediculosis pubis in the pubic area, caused by *Pithirus pubis;* (2) Pediculosis corporis on the trunk, caused by *Pediculus humanus humanus;* and (3) Pediculosis capitis on the head, caused by *Pediculus humanis capitis.* Pediculosis affects millions of humans world-wide, causing itching and discomfort.

Generally, infestation is treated by application of a fluid composition that kills the lice by poisoning (e.g., compositions including pesticides), or by physical means (smothering, dehydration).

One type of non-toxic composition for treating lice infestation includes a combination of silicone oil and isopropyl myristate, for example commercially available as Resultz® (50% isopropyl myristate and 50% cyclomethicone) or Full Marks Solution (SSL Healthcare Ireland Ltd, Dublin, Ireland). Such compositions kill adult lice through two modes of action. The isopropyl myristate is a pediculicide which dissolves the waxy coating of the louse exoskeleton, leading to dehydration of the louse, while the silicone oil, which is a glidant, makes it difficult for weakened lice to grasp the hair, such that the lice are easy to comb out from the hair. Additionally, silicone oil has some lice choking effect. For use, the composition is carefully applied to the scalp and the entire length of the hair shafts, maintained in contact therewith for at least 10 minutes followed by washing, rinsing and combing with a fine-tooth comb to remove dead lice.

Meinking et al. (Ped Dermatol, vol. 27(1), 2010, pages 19-24 describes clinical trials supporting use of benzyl alcohol lotion 5% for topical treatment of head lice.

A disadvantage of such compositions is that these lack efficacy against nits, the eggs of lice that are attached to the hair with a waterproof adhesive. Additionally, subsequent to use, some such compositions leave an unpleasant residue that is not easily washed out.

It is desirable to have a composition for the treatment of lice that has some advantage over known treatments.

### SUMMARY OF THE INVENTION

Some embodiments of the invention relate to compositions for the treatment of infestations by insects, especially infestations by lice and nits (pediculosis).

Aspects and embodiments of the invention are described in the specification hereinbelow and in the appended claims.

According to the invention, there is provided a composition comprising at least 0.8% by weight.benzyl alcohol, at least 4% by weight diacetone alcohol, and at least 2.5% by weight triethyl citrate, for use in treating pediculosis.

According to some embodiments, the composition further comprises acetic acid.

According to some embodiments, the composition further comprises a hair wetter, such as a hair wetter having a spreading coefficient of at least about 1100 mm/10 minutes, (for example, at least one of isopropyl myristate, dicaprylyl ether, dicaprylyl carbonate, isopropyl palmitate, C₁₂₋₁₅ alkyl benzoate, isopropyl isostearate, butyl stearate, diisopropyl adipate, diisobutyl adipate, diethylhexyl adipate, butyl myristate, hexyl myristate, tributyl citrate, acetyltributyl citrate, acetyltriethyl citrate, ethylhexyl cocoate, ethylhexyl stearate, ethylhexyl pelargonate, ethylhexyl succinate, ethylhexyl cocoate, ethyl oleate, propylene glycol dicaprylate/caprate, propylheptyl caprylate, coco-caprylate, and PPG-2 myristyl ether propionate, or combinations thereof). According to some embodiments, the hair wetter comprises isopropyl myristate.

According to some embodiments, the composition further comprises at least one humectant selected from the group consisting of glycerin, glycerol, propylene glycol, glyceryl triacetate, a sugar polyol, sorbitol, xylitol, maltitol, a polymeric polyol, polydextrose, quillaia, lactic acid, urea and combinations thereof. In some such embodiments, the composition comprises at least 1%, at least 2% or at least 2.5% by weight of humectant.

According to some embodiments, the composition further comprises a pharmaceutically acceptable carrier.

As used herein, the term "pharmaceutically acceptable" means approved by a regulatory agency of the US Federal or a US state government or listed in the U.S. Pharmacopeia or other generally recognized pharmacopeia for use in animals, and more particularly in humans. Herein, the phrase "pharmaceutically acceptable carrier" refers to an approved carrier or a diluent that does not cause significant irritation to an organism and does not abrogate the biological activity and properties of the administered conjugate.

As used herein, the term "carrier" refers to a diluent, adjuvant, excipient, or vehicle with which the therapeutic is administered.

Unless otherwise defined, all technical and scientific terms used herein have the same meaning as commonly understood by one of ordinary skill in the art to which the invention pertains. In case of conflict, the specification, including definitions, takes precedence.

As used herein, the term "treating" includes curing a condition, treating a condition, preventing a condition, treating symptoms of a condition, curing symptoms of a condition, ameliorating symptoms of a condition, treating effects of a condition, ameliorating effects of a condition, and preventing results of a condition.

Herein the term "excipient" refers to an inert substance added to a pharmaceutical composition to further facilitate processes and administration of the active pharmaceutical ingredients.

As used herein, the terms "comprising", "including", "having" and grammatical variants thereof are to be taken as specifying the stated features, integers, steps or components but do not preclude the addition of one or more additional features, integers, steps, components or groups thereof. These terms encompass the terms "consisting of' and "consisting essentially of'.

As used herein, the indefinite articles "a" and "an" mean "at least one" or "one or more" unless the context clearly dictates otherwise.

### DESCRIPTION OF SOME EMBODIMENTS OF THE INVENTION

The invention, in some embodiments, relates to the field of pest control, and more specifically, but not exclusively, to compositions for treating infestations by insects, especially infestations by lice (pediculosis).

The principles, uses and implementations of the teachings herein may be better understood with reference to the accompanying description. Upon perusal of the description present herein, one skilled in the art is able to implement the invention without undue effort or experimentation.

Before explaining at least one embodiment in detail, it is to be understood that the invention is not necessarily limited in its application to the details of construction and the arrangement of the components and/or methods set forth herein. The invention is capable of other embodiments or of being practiced or carried out in various ways. The phraseology and terminology employed herein are for descriptive purpose and should not be regarded as limiting.

The present invention, in at least some embodiments provides a composition comprising at least 0.8% by weight benzyl alcohol, at least 4% by weight diacetone alcohol, and at least 2.5% by weight triethyl citrate, for use in treating pediculosis. In some embodiments, the solvent further comprises acetic acid.

In some embodiments, the composition comprises from 4 to 4.5% by weight diacetone alcohol.

In some embodiments, the composition comprises 3% by weight triethyl citrate.

In some embodiments, the composition comprises 1% by weight benzyl alcohol. In some embodiments, the benzyl alcohol also serves as a preservative.

In some embodiments, a composition includes at least one humectant selected from the group consisting of glycerin; glycerol; propylene glycol; glyceryl triacetate; sugar polyols such as sorbitol, xylitol and maltitol; polymeric polyols such as polydextrose; quillaia, lactic acid or urea, or combinations thereof. In some embodiments, a humectant comprises glycerin. In some embodiments, the composition comprises at least 1%, at least 2% and even at least 2.5% by weight of humectant. In some embodiments, the composition comprises 2.5 to 3% by weight of glycerin.

According to some embodiments, the composition disclosed herein is for dissolving nit glue.

Nit glue is a specialized saliva secreted by lice in order to attach nits (lice eggs) to the hair of a host, forming a very strong bond. Nits which remain in the hair may remain viable after elimination of adult lice, resulting in hatching of fresh nymphs, resulting in re-infestation even after all live lice have been removed.

In some embodiments, the composition further comprises a hair wetter i.e. an oil or solvent which spreads quickly over the hair.

In some embodiments, the hair wetter has a spreading coefficient of at least 1200 mm/10 minutes. In some embodiments, the spreading coefficient is at least 1250 mm /10 minutes, at least 1400 mm/10 minutes, at least 1450 mm/10 minutes, at least 1560 mm/10 minutes, at least 1600 mm/10 minutes, at least 1700 mm/10 minutes, and even at least 1840 mm/10 minutes.

Examples of suitable hair wetters include, without limitation, isopropyl myristate, dicaprylyl ether, dicaprylyl carbonate, isopropyl palmitate, C₁₂₋₁₅ alkyl benzoate, isopropyl isostearate, butyl stearate, diisopropyl adipate, diisobutyl adipate, diethylhexyl adipate, butyl myristate, hexyl myristate, tributyl citrate, acetyltributyl citrate, acetyltriethyl citrate, ethylhexyl cocoate, ethylhexyl stearate, ethylhexyl pelargonate, ethylhexyl succinate, ethylhexyl cocoate, ethyl oleate, propylene glycol dicaprylate/caprate, propylheptyl caprylate, coco-caprylate, and PPG-2 myristyl ether propionate, or combinations thereof.

A list of exemplary hair wetters and their respective spreading coefficients is provided in Table 1.

**TABLE 1: Hair Wetters**

| **Hair wetter/Emollient/Solvent** | **CAS Number** | **Spreading coefficient mm/10 minutes** |
|---|---|---|
| Dicaprylyl Ether | 629-82-3 | 1600 |
| Dicaprylyl Carbonate | 1680-31-5 | 1600 |
| Isopropyl Myristate | 110-27-0 | 1200 |
| Diisopropyl Adipate | 6938-94-9 | 1840 |
| Isopropyl Palmitate | 142-91-6 | 1450 |
| Diisobutyl Adipate | 141-04-8 | 1990 |
| Ethylhexyl Cocoate | 92044-87-6 | 1470 |
| Diethylhexyl Adipate | 103-23-1 | 1430 |
| Ethyl Oleate | 111-62-6 | 1850 |
| Butyl Myristate | 64131-21-1 | 1700 |
| Isopropyl Isostearate | 68171-33-5 | 1250 |
| Butyl Stearate | 123-95-5 | 1660 |
| C12-15 Alkyl Benzoate | 68411-27-8 | 1360 |
| Propylheptyl Caprylate | 868839-23-0 | 1900 |
| Coco-Caprylate | 107525-85-9 | 1300 |
| PPG-2 Myristyl Ether Propionate | 74775-06-7 | 1600 |
| Ethylhexyl Pelargonate | 59587-44-9 | 1670 |
| Ethylhexyl Stearate | 22047-49-0 | 1400 |
| Ethylhexyl Succinate | 2915-57-3 | 1560 |
| Propylene Glycol Dicaprylate/Caprate | 68583-51-7 | 1700 |

According to a preferred embodiment, the hair wetter comprises isopropyl myristate, which, in addition to serving as a hair wetter, has pediculocidic properties.

The compositions described herein may be manufactured using processes well known in the art, e.g., by means of conventional mixing, dissolving, emulsifying etc. Further techniques for formulation and administration of active ingredients may be found in "Remington's Pharmaceutical Sciences," Mack Publishing Co., Easton, PA, latest edition.

In some embodiments, a composition as described herein further comprises a suitable carrier to facilitate administration.

In some embodiments, a composition as described herein further comprises additional excipients and auxiliaries, known to those skilled in the art, to facilitate processing of the active ingredients into preparations which, can be used pharmaceutically. Non-limiting examples of such excipients include anti-oxidants, gelling agents, thickening agents, stabilizers, and the like.

A composition as described herein may be used for treating any subject suffering from pediculosis, either a human or a non-human animal. A composition as described herein may be used in any suitable manner, but is preferably applied topically to the subject, most preferably to the skin and/or hair and/or pelt and/or fur of the subject. In some embodiments, the composition is used in a manner analogous to the use of "Hairclean 1-2-3"® or Resultz®.

For topical administration, a composition as described herein is provided in any suitable form which enables the composition to remain in contact with the skin and/or hair for a desired time period. Non-limiting examples of suitable forms include a liquid, a gel, a cream, an ointment, a paste, a lotion, a milk, a suspension, an aerosol, a spray, a foam, or a serum.

In some embodiments, a composition as described herein is applied (e.g., by massaging into the hair) topically for a period of time up to 15 minutes, up to 10 minutes, and even up to 5 minutes. In some embodiments, the composition is applied for 5 minutes.

After the period of time, the hair is combed with a fine-tooth comb to remove lice.

In some embodiments, after the composition has been left in contact with the skin and/or hair of the subject, the composition is removed either by rinsing with water or by washing with normal shampoo followed by rinsing.

Exemplary embodiments are discussed hereinbelow with reference to specific materials, methods and examples. The material, methods and examples discussed herein are illustrative and not intended to be limiting. In some embodiments, methods and materials similar or equivalent to those described herein are used in the practice or testing of embodiments of the invention. It is to be understood that the invention is not necessarily limited in its application to the details of construction and the arrangement of the components and/or methods set forth in the following description and/or illustrated in the drawings. The invention is capable of other embodiments or of being practiced or carried out in various ways.

### EXPERIMENTAL

### Example 1: Exemplary compositions

Three exemplary compositions that are embodiments of the teachings herein were prepared in accordance with the teachings herein using commercially-available ingredients are listed in Table 2:

**Table 2: Components of compositions I, II and III**

| *Component* | *(%w*/*w)* | *(% w*/*w)* | *(% w*/*w)* | |
|---|---|---|---|---|
| | | | | |
| D.I. Water | 45.4 | 48.4 | 47.15 | solvent |
| Isopropanol | 4.5 | 4.0 | 4.7 | non aqueous solvent |
| | | | | |
| Isopropyl myristate | 5.7 | 5.5 | 6.0 | Hair lubricant / pediculicide |
| Cyclomethicone (*CM-040 and*/*or DC345*) | 7.0 | 7.0 | 6.5 | Hair lubricant / pediculicide |
| Polyquaternium-3 7 (*Ultragel*® *300*) | 1.5 | 1.0 | 1.0 | Hair Lubricant / conditioner Thickener, Stabilizer |
| Cyclopentasiloxane and/or Dimethiconol (*Belsil*® *CM1000 and.*/*or DC 1501 and*/*or SF1202*) | 0.5 | 0.5 | 0.6 | Hair lubricant / conditioner / pediculicide |
| | | | | |
| Cetyl PEG/PPG-10/1 Dimethicone (*Abil*® *EM90)* | 0.5 | 0.5 | 0.6 | water in oil emulsifier, hair lubricant, conditioner |
| Cetearyl Isononanoate, Ceteareth-20, Cetearyl Alcohol, Glyceryl Stearate, Glycerin, Ceteareth-12, Cetyl Palmitate (*Emulgade*® *CM*) | 14.0 | 14.0 | 14.0 | oil-in-water emulsion mixture containing oil-in-water emulsifiers, emollients, wax, oil-in-water coemulsifiers, oily waxes and humectants |
| Polyacrylate-1 Crosspolymer (*Carbopol*® *Aqua CC*) | 2.0 | 2.0 | 0.1 | thickener, stabilizer |
| PEG-150/Decyl Alcohol/SMDI Copolymer (*Aculyn*™ *44*) | 0 | 0 | 2.7 | thickener, stabilizer |
| | | | | |
| Diacetone Alcohol | 4.5 | 4.0 | 4.7 | Nit glue dissolver |
| Glacial acetic acid | 4.8 | 4.0 | 5.0 | Nit glue dissolver, pH adjustor |
| Triethyl Citrate | 3.0 | 3.0 | 3.0 | Nit glue dissolver |
| Benzyl alcohol | 1.0 | 1.0 | 1.0 | Nit glue dissolver /preservative |
| | | | | |
| Glycerin | 3 | 2.5 | 1.0 | humectant |
| | | | | |
| Lvchee-Melon Fragrance | 1.5 | 1.5 | 1.8 | fragrance |
| Orange Peel Fragrance | 1.0 | 1.0 | 0.1 | fragrance |
| | | | | |
| Triethanolamine | qs (0.1) | qs (0.1) | qs (0.05) | pH adjustor |

### Materials:

The required materials were purchased from commercial suppliers:
isopropanol (CAS 67-63-0), diacetone alcohol (CAS 123-42-2) and glycerin (CAS 56-81-5) from Gadot Lab Supplies (Or Akiva, Israel);
isopropyl myristate (CAS 110-27-0) and Polyquaternium-37 (Ultragel® 300, CAS 26161-33-1) from Cognis (Monheim, Germany a subsidiary of BASF SE, Ludwigshafen, Germany);
cyclomethicone was CM-040 from Wacker Chemie AG (Munich, Germany) and/or DC345 from Dow Corning Corporation (Midland, Michigan, USA);
cyclopentasiloxane and/or dimethiconol for Compositions I and II was DC 1501 from Dow Corning Corporation and/or Belsil® CM 1000 from Wacker Chemie AG (München, Germany), and for Composition III was SF1202 from Momentive Performance Materials Inc. (Albany, New York, USA);
a blend of Cetearyl Isononanoate, Ceteareth-20, Cetearyl Alcohol, Glyceryl Stearate, Glycerin, Ceteareth-12 and Cetyl Palmitate (CAS Numbers: 84878-33-1, 84878-34-2, 68439-49-6, 67762-27-0, 67701-33-1, 56-81-5, 95912-87-1, 68439-49-6) was Emulgade® CM from Cognis part of BASF SE (Ludwigshafen, Germany);
cetyl PEG/PPG-10/1 dimethicone was Abil® EM90 (CAS 145686-34-6) from Evonik Goldschmidt GmbH (Essen, Germany);
polyacrylate-1 crosspolymer was Carbopol® Aqua CC from Lubrizol Corporation (Wickliffe, Ohio, USA);
glacial acetic acid (CAS 64-19-7) from Riedel-de Haën (Seelze, Germany);
triethyl citrate (CAS 77-93-0) and benzyl alcohol (CAS 100-51-6) from Sharon Laboratories (Ashdod, Israel);
lychee-melon fragrance and orange peel fragrance from S.M. Naturals (North Richland, Texas, USA);
triethanolamine (CAS 102-71-6) from Merck KgGA (Darmstadt, Germany); and
PEG-150/Decyl Alcohol/SMDI Copolymer was Aculyn™ 44 from Rohm and Haas (Philadelphia, Pennsylvania, USA) a subsidiary of Dow Chemical (Midland, Michigan, USA).

### Example 2: Preparation of an exemplary composition

A clean mixer equipped with agitating planetary propeller and high shear homogenizer or dispersing disc was prepared. Diacetone alcohol, isopropyl alcohol, acetic acid, glycerin, benzyl alcohol, triethyl citrate and fragrances were added to the mixer at room temperature, with stirring for 5 minutes.

In an auxiliary vessel, a mixture of Polyquaternium-37 in water was prepared by mixing at room temperature until a clear gel was obtained. The mixture was checked to ensure complete dissolution of the Polyquaternium-37. The clear gel from the auxiliary vessel was added to the mixer while mixing. The contents of the mixer were mixed for an additional 5 minutes.

Cetearyl isononanoate (and) Ceteareth-20 (and) cetearyl alcohol (and) glyceryl stearate (and) glycerin (and) Ceteareth-12 (and) cetyl palmitate, isopropyl myristate, and cyclopentasiloxane, were added to the mixer, at room temperature, while mixing, and stirred for 5 minutes. Mixing was continued until a smooth, white, emulsion was obtained.

Cyclopentasiloxane (and) dimethiconol, and cetyl PEG/PPG-10/1 dimethicone were added while applying high shear homogenizer or high rpm dispersing disc. Mixing was continued until a smooth, white, gel-emulsion was obtained

Polyacrylate-1 Crosspolymer (and in Compositions II and III, also PEG-150/Decyl Alcohol/SMDI Copolymer) was added to the mixer, at room temperature, while mixing. Mixing was continued until a smooth, white, gel-cream was obtained. The pH of the mixture was adjusted from 2.4 to 3.2 by adding triethanolamine.

The resulting composition was a homogenous white-colored emulsion.

### Example 3: Pediculicidal activity of the exemplary compositions

The pediculicidal and ovicidal activities of exemplary compositions are tested in the laboratory on lice according to the following procedure:
Body lice (*Pediculus humanus humanus*) are reared in the laboratory according to the method described by Cole M.M. [Body lice. In: Insect colonization and mass production. Smith, C.N. (ed.), Academic Press, N.York, p. 15-24 (1966)].

For each test, 50 lice (10 males, 10 females and 30 nymphs) are placed on a 7-cm white filter paper disc and exposed to 1 gram of an exemplary composition. The lice are left in contact with the composition for 5 to 15 minutes. The lice are then removed from the filter paper, washed with normal shampoo (1:20) for 1 minute and then with tap water for 1 minute. After treatment, lice are transferred to a fresh filter paper disc and incubated overnight at optimum temperature and humidity. Mortality is determined after 24 hours. As a negative control, lice are treated with an isopropanol or 40% ethanol / 60% water solution. Each exposure time is tested in triplicate.

### Example 4: Ovicidal activity of the exemplary compositions

Lice are placed on human hair every other day for 5 days and left for 24 to 48 hrs for oviposition. Fifty nits (lice eggs) 2 to 6 days old, are treated with the exemplary compositions, as described above. The number of hatched and non-hatched nits are counted after 10 days. Each exposure time is tested in triplicate.

### Example 5: Pediculicidal activity of composition III

The pediculicidal and ovicidal activities of composition III were tested in the laboratory on lice according to the following procedure.

Body lice (*Pediculus humanus humanus*) were reared in the laboratory according to the method described by Cole M.M. [Body lice. In: Insect colonization and mass production. Smith, C.N. (ed.), Academic Press, New York, NY, USA p. 15-24 (1966)].

For each test, 50 lice (10 males, 10 females and 30 nymphs) were placed on a 7-cm white filter paper disc and exposed to 1 gram of composition III. The lice were left in contact with the composition for 5 to 15 minutes. The lice were then removed from the filter paper, washed with normal shampoo (1:20) for 1 minute and then with tap water for 1 minute. After treatment, the lice were transferred to a fresh filter paper disc and incubated overnight at optimum temperature and humidity. Mortality was determined after 24 hours. As a negative control, lice were treated with a solution of 40% ethanol / 60% water. Each exposure time was tested in triplicate. The results are listed in Table 3.

### Example 6: Ovicidal activity of composition III

Lice were placed on human hair every other day for 5 days and left for 24 to 48 hours for oviposition. Fifty nits, 2 to 6 days old, were treated with composition III, as described above in Example 3. The number of hatched and non-hatched nits were counted after 10 days. Each exposure time was tested in triplicate. The results are listed in Table 3.

**Table 3: Pediculicidal and Ovicidal activity of composition III**

| **Composition** | **Exposure time [min]** | **Example 5 Mean lice mortality [%]** | **Example 6 Mean nit mortality [%]** |
|---|---|---|---|
| **III** | 5 | 100 | 100 |
| **III** | 10 | 100 | 100 |
| **III** | 15 | 100 | 100 |
| **Control** | 15 | 12 | 16 |

From Table 3 is seen that composition III, an embodiment of the composition described herein, shows significant pediculicidal and ovicidal activity, after exposure times even at least as short as 5 minutes.

### Example 7: Clinical study of composition III

To study the clinical efficacy, as well as ease of use and tolerability, of composition III, 30 human children aged 5 to 15 years (average age: 8.8 years, 82% females, 75% having long hair) were treated with a single application of composition III.

### Before application of composition III

The hair of the children was examined by a qualified nurse. It was ascertained that the hair of 89% of the children was infested with lice (mean: 30 lice) and nits (mean: 150 nits). 15% of the children had greater than 200 lice.

### Application of composition III

A child's hair was dampened. Composition III was applied to the damp hair of the child, and massaged-in to ensure that the composition contacted substantially all the hair and scalp. A qualified nurse combed a section of the hair with a louse-comb in the usual way at 5, 10, 15 and 20 minutes after application of the composition. After the combing subsequent to 20 minutes from application of the composition, the child's hair was rinsed with water and the qualified nurse thoroughly combed the hair with a louse-comb in the usual way. After another 7 days, a qualified nurse thoroughly combed the child's hair with a louse-comb in the usual way.

### Five minutes after application of composition III

Five minutes after application of composition III, a first section of the child's hair was combed with a louse-comb. In 80% of the children, only dead lice and no living lice were recovered from the louse-comb. In 70% of the children, the nits were easily removed with the louse-comb from the first section of the hair, indicating that the composition had dissolved the nit glue.

### Ten minutes after application of composition III

Ten minutes after application of composition III, a second section of the hair (different from the first section) of the child's hair was combed with a louse-comb. In all of the children, only dead lice and no living lice were recovered from the louse-comb. In 90% of the children, the nits were easily removed with the louse-comb from the second section of the hair, indicating that the composition had dissolved the nit glue.

### Fifteen and twenty minutes after application of composition III

Fifteen and twenty minutes after application of composition III, no living lice were recovered using the louse comb from the hair of any of the children and nits were easily removed from the hair of all of the children.

### After rinsing and brushing

After the composition was rinsed from a child's hair, the hair was brushed and combed using a louse-comb in the usual way. No substantial residue of the composition was observed in the hair of any of the children. Subsequently, the hair was thoroughly combed with a louse-comb. No dead lice were recovered from the louse-comb of 80% of the children, and an average of 2 dead lice were recovered from the louse comb of 20% of the children. An average of 30 nits were recovered from the louse-comb.

### After seven days

Seven days after the treatment, a few nits were found in the hair of some of the children. In the hair of two of the children who previously had more than 200 living lice, a few living nymphs were found.

### Conclusions

Composition III was well-tolerated (including acceptable scent), easy to apply and no adverse reactions were observed. After rinsing of the hair, no substantial residue of compositions was noted in the hair.

The composition was found to be highly effective in treating lice infestation even at short application times. The presence of several living nymphs in some children indicate that it may be advisable to repeat treatment after about 7 days in cases of exceptionally serious infestation.

## Claims

1. A composition comprising:
at least 0.8% by weight benzyl alcohol,
at least 4% by weight diacetone alcohol, and
at least 2.5% by weight triethyl citrate
for use in treating pediculosis.

2. The composition of claim 1, further comprising acetic acid.

3. The composition of any one of claims 1 to 2, further comprising a hair wetter, optionally isopropyl myristate.

4. The composition of claim 3, wherein said hair wetter has a spreading coefficient of at least 1100 mm/10 minutes.

5. The composition of claim 3, wherein said hair wetter is selected from the group consisting of isopropyl myristate, dicaprylyl ether, dicaprylyl carbonate, isopropyl palmitate, C12-15 alkyl benzoate, isopropyl isostearate, butyl stearate, diisopropyl adipate, diisobutyl adipate, diethylhexyl adipate, butyl myristate, hexyl myristate, tributyl citrate, acetyltributyl citrate, acetyltriethyl citrate, ethylhexyl cocoate, ethylhexyl stearate, ethylhexyl pelargonate, ethylhexyl succinate, ethyl oleate, propylene glycol dicaprylate/caprate, propylheptyl caprylate, coco-carpylate, and PPG-2 myristyl ether propionate, or combinations thereof.

6. The composition of any one of claims 1 to 5, further comprising at least one humectant selected from the group consisting of glycerin, glycerol, propylene glycol, glyceryl triacetate, a sugar polyol, sorbitol, xylitol, maltitol, a polymeric polyol, polydextrose, quillaia, lactic acid, urea and combinations thereof.

7. The composition of claim 6, the composition comprising at least 1% by weight said humectant.

8. The composition of claim 6, the composition comprising at least 2% by weight said humectant.

9. The composition of claim 6, the composition comprising at least 2.5% by weight said humectant.

## Patentansprüche

1. Zusammensetzung, die Folgendes umfasst:
mindestens 0,8 Gew.-% Benzylalkohol, mindestens 4 Gew.-% Diacetonalkohol und mindestens 2,5 Gew.-% Triethylcitrat zur Verwendung in der Behandlung von Pedikulose.

2. Zusammensetzung nach Anspruch 1, ferner umfassend Essigsäure.

3. Zusammensetzung nach einem der Ansprüche 1 bis 2, ferner umfassend einen Haarbenetzer, optional Isopropylmyristat.

4. Zusammensetzung nach Anspruch 3, wobei der Haarbenetzer einen Spreitparameter von mindestens 1100 mm/10 Minuten aufweist.

5. Zusammensetzung nach Anspruch 3, wobei der Haarbenetzer ausgewählt ist aus der Gruppe bestehend aus Isopropylmyristat, Dicaprylylether, Dicaprylylcarbonat, Isopropylpalmitat, C12-15-Alkylbenzoat, Isopropylisostearat, Butylstearat, Diisopropyladipat, Diisobutyladipat, Diethylhexyladipat, Butylmyristat, Hexylmyristat, Tributylcitrat, Acetyltributylcitrat, Acetyltriethylcitrat, Ethylhexylcocoat, Ethylhexylstearat, Ethylhexylpelargonat, Ethylhexylsuccinat, Ethyloleat, Propylenglycoldicaprylat/-caprat, Propylheptylcaprylat, Cococaprylat und PPG-2-Myristyletherproprionat oder Kombinationen davon.

6. Zusammensetzung nach einem der Ansprüche 1 bis 5, ferner umfassend mindestens ein Feuchthaltemittel ausgewählt aus der Gruppe bestehend aus Glycerin, Glycerol, Propylenglycol, Glyceryltriacetat, einem Zuckerpolyol, Sorbitol, Xylitol, Maltitol, einem polymeren Polyol, Polydextrose, Quillaia, Milchsäure, Urea und Kombinationen davon.

7. Zusammensetzung nach Anspruch 6, wobei die Zusammensetzung mindestens 1 Gew.-% des Feuchthaltemittels umfasst.

8. Zusammensetzung nach Anspruch 6, wobei die Zusammensetzung mindestens 2 Gew.-% des Feuchthaltemittels umfasst.

9. Zusammensetzung nach Anspruch 6, wobei die Zusammensetzung mindestens 2,5 Gew.-% des Feuchthaltemittels umfasst.

## Revendications

1. Composition comprenant :
au moins 0,8 % en poids d'alcool benzylique, au moins 4 % en poids d'alcool diacétonique et au moins 2,5 % en poids de citrate de triéthyle pour utilisation dans le traitement de la pédiculose.

2. Composition selon la revendication 1, comprenant en outre de l'acide acétique.

3. Composition selon l'une quelconque des revendications 1 et 2, comprenant en outre un agent mouillant pour cheveux, éventuellement du myristate d'isopropyle.

4. Composition selon la revendication 3, dans laquelle ledit agent mouillant pour cheveux a un coefficient d'étalement d'au moins 1100 mm/10 minutes.

5. Composition selon la revendication 3, dans laquelle ledit agent mouillant pour cheveux est sélectionné parmi le groupe constitué du myristate d'isopropyle, de l'éther de dicaprylyle, du carbonate de dicaprylyle, du palmitate d'isopropyle, du benzoate d'alkyle de C₁₂ à C₁₅, de l'isostéarate d'isopropyle, du stéarate de butyle, de l'adipate de diisopropyle, de l'adipate de diisobutyle, de l'adipate de diéthylhexyle, du myristate de butyle, du myristate d'hexyle, du citrate de tributyle, du citrate d'acétyltributyle, du citrate d'acétyltriéthyle, du cocoate d'éthylhexyle, du stéarate d'éthylhexyle, du pélargonate d'éthylhexyle, du succinate d'éthylhexyle, de l'oléate d'éthyle, du dicaprylate/caprate de propylèneglycol, du caprylate de propylheptyle, du cococarpylate et du propionate d'éther myristique de PPG-2 ou de combinaisons de ceux-ci.

6. Composition selon l'une quelconque des revendications 1 à 5, comprenant en outre au moins un humectant sélectionné dans le groupe constitué de la glycérine, du glycérol, du propylèneglycol, du triacétate de glycéryle, d'un polyol, du sorbitol, du xylitol, du maltitol, d'un polyol polymère, d'un polydextrose, d'une quillaia, de l'acide lactique, de l'urée et de combinaisons de ceux-ci.

7. Composition selon la revendication 6, la composition comprenant au moins 1 % en poids dudit humectant.

8. Composition selon la revendication 6, la composition comprenant au moins 2 % en poids dudit humectant.

9. Composition selon la revendication 6, la composition comprenant au moins 2,5 % en poids dudit humectant.
